# EUROPEAN PATENT APPLICATION

(11) **EP 3 158 993 A1**
(43) Date of publication of application: **26.04.2017**
(21) Application number: 15460098.5
(22) Date of filing: 22.10.2015
(51) Int. Cl.: A61K 9/20, A61K 31/4365

(54) **PROCESS FOR PREPARING A TABLET COMPRISING PRASUGREL OR PHARMACEUTICALLY ACCEPTABLE SALT THEREOF**

(71) Applicant: Zaklady Farmaceutyczne Polpharma SA, 83-200 Starogard Gdanski (PL)
(72) Inventor: Przerada, Szymon, 83-200 Starogard Gdanski (PL); Okularczyk, Marcin, 83-212 Dabrówka (PL)
(74) Representative: Obrycki, Pawel Andrzej

(57) **Abstract**

The present invention relates to a process for preparing a tablet preferably for oral administration comprising prasugrel or pharmaceutically acceptable salt thereof.

## Description

### FIELD OF THE INVENTION

The present invention relates to a process for preparing a tablet preferably for oral administration comprising prasugrel or pharmaceutically acceptable salt thereof.

### STATE OF THE ART

Prasugrel is a prodrug belonging to the group of thienopyridine derivatives and is an adenosine diphosphate (ADP) receptor antagonist. It is a potent inhibitor of ADP-mediated platelet aggregation in vivo and is approved for use in combination with acetylsalicylic acid for the treatment or prophylaxis of thrombosis or thromboembolism and related diseases.

Prasugrel is the INN (International Non-proprietary Name) of a compound having the systematic chemical name 2-acetoxy-5-(alpha-cyclopropylcarbonyl-2-fluorobenzyl)-4,5,6,7-tetrahydrothieno[3,2-c]pyridine, a molecular formula C₂₀H₂₀FNO₃S, and the following structural Formula I.

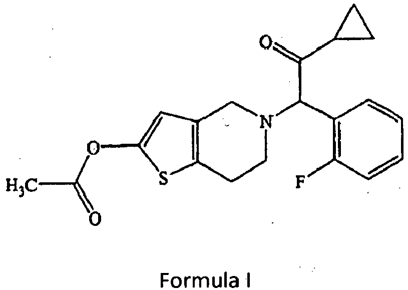

Prasugrel, together with other tetrahydrothienopyridine derivatives, has been described for the first time in the patent application EP 542411. That patent document discloses the use of these compounds for treatment and prophylaxis of thrombosis and embolism.

Prasugrel pharmaceutically acceptable salts including hydrochloride and maleate have been described in the international patent application WO 02/04461. The hydrochloride salt of prasugrel is the active ingredient used in Efient^{®}, a drug product marketed by Daiichi-Sankyo and Eli Lilly. Additionally, prasugrel hydrobromide and its crystalline forms have been described in the international patent application WO 2011/127300.

Prasugrel and its salts are subject to facile hydrolysis and oxidation, leading to stability problems of the active ingredient, both during manufacturing and storage of the formulated drug.

International patent application WO 2006/135605 describes a tablet comprising a therapeutically effective amount of prasugrel hydrochloride packed in an air and moisture impervious blister package under an inert gas to improve the stability and shelf life of prasugrel. The exemplary formulation disclosed in the application comprises prasugrel hydrochloride, mannitol, hydroxypropyl methylcellulose, croscarmellose sodium, microcrystalline cellulose and magnesium stearate.

Patent application US 2009/0281136 relates to a stable pharmaceutical formulation comprising prasugrel or pharmaceutically acceptable salt thereof and discloses a pharmaceutical formulation comprising prasugrel or pharmaceutically acceptable salt thereof and at least one stabilizing agent which comprises at least one of an antioxidant and a buffer.

Patent application EP 2275087A discloses the manufacturing process of embedding sensitive particles of prasugrel base in different protective low-melting matrices under mild conditions.

Taking into account the above solutions, there is still a need to provide a process of obtaining stable tablet comprising prasugrel or pharmaceutically acceptable salt thereof. That process should be as simple as possible without involving the use of any special excipients such as antioxidants and special manufacturing procedures such as melt granulation.

### SUMMARY OF THE INVENTION

Surprisingly, it was found that a stable tablet comprising prasugrel or pharmaceutically acceptable salt thereof and one or more pharmaceutically acceptable excipients can be obtained by applying process of tablet conditioning at a temperature above 40°C. Such tablet is more stable than tablet obtained without the step of tablet conditioning at a temperature above 40°C.

### DETAILED DESCRIPTION OF THE INVENTION

The present invention relates to a process for preparing a tablet preferably for oral administration comprising prasugrel or pharmaceutically acceptable salt thereof and one or more pharmaceutically acceptable excipients while the process comprises the step of tablet, conditioning at a temperature above 40°C. Preferably the step of tablet conditioning is performed at a temperature from 45°C to 70°C, more preferably from 50°C to 65°C, most preferably from 50°C to 60°C. Preferably the duration of the step of tablet conditioning ranges from 5 minutes to 3 hours, more preferably from 10 minutes to 2 hours, most preferably from 15 minutes to 1 hour.

Process of the present invention applies to a tablet comprising prasugrel or pharmaceutically acceptable salt thereof, preferably prasugrel base, prasugrel hydrochloride, prasugrel hydrobromide or prasugrel maleate, more preferably prasugrel hydrochloride, prasugrel hydrobromide or prasugrel maleate, most preferably prasugrel hydrochloride or prasugrel maleate. In case of the hydrochloride salt preferably crystalline form B1 or B2 is used, most preferably form B2, as described in EP 1298132 A1 (Example 3 and Example 4) and in case of the maleate salt preferably the crystalline form as described in EP 1298132 A1 (Example 5) is used. Preferably the prasugrel form used in the tablet of the process of the present invention is micronized to PSD d(0.9) below 15 µm, preferably below 12 µm, most preferably below 10 µm, as measured with a use of laser diffraction method with the following conditions:
Particle size analyzer: Mastersizer 2000, manufactured by Malvern Instruments Dispersion unit: Hydro 2000S (wet dispersion)

| | |
|---|---|
| Analysis model: | general purpose |
| Sensitivity: | normal |
| Dispersant: | sunflower oil |
| Dispersant refractive index: | 1.47 |
| Sample refractive index: | 1.7 |
| Sample absorption index: | 0.1 |
| Single measurement time: | 5 s |
| Background measurement time: | 10 s |
| Stirrer speed: | 2000 rpm |
| Obscuration: | within 10 - 25% |
| External ultrasounds: | 1 min |

Preferably the process of the present invention applies to a tablet comprising apart from prasugrel or a salt thereof one or more pharmaceutically acceptable excipients, wherein at least one of said excipients is selected from the group consisting of glycerol dibehenate, glycerol monostearate, glycerol palmitostearate and a mixture thereof, preferably from the group consisting of glycerol dibehenate, glycerol palmitostearate and mixture thereof, in particular said excipient being glycerol dibehenate. Preferably glycerol dibehenate, glycerol monostearate, glycerol palmitostearate or a mixture thereof is used as a lubricant. Preferably glycerol dibehenate, glycerol monostearate, glycerol palmitostearate or mixture thereof are used in an amount of 0.1% to 15%, more preferably 0.2% to 8%, most preferably 0.5% to 5% based on the total weight of the uncoated tablet. Preferably the process of the present invention applies to a tablet comprising one or more further pharmaceutically acceptable excipients which are selected from the group consisting of binder, filler, diluent, disintergant, glidant and/or lubricant. The glidant and the lubricant in some cases are used interchangeably as they often can exhibit both properties. Preferably the suitable binders, fillers, diluents, disintegrants, glidants and lubricants are those known and described in the Handbook of Pharmaceutical Excipients, seventh edition (2012).

Preferably the process of the present invention applies to a tablet which is prepared by technics known in the art, preferably dry granulation, wet granulation or direct compression, more preferably dry granulation or direct compression, most preferably direct compression.

Preferably the step of tablet conditioning is performed after tablet coating. Preferably coating of the tablet is cosmetic or a functional coating with the use of a coating agent known in the art, more preferably it is a cosmetic coating. Preferably the agents described in the Handbook of Pharmaceutical Excipients, seventh edition (2012) are to be applied as the coating agent.

Preferably the lubricant is a mixture of any known lubricant with a lubricant selected from the group consisting of glycerol dibehenate, glycerol monostearate, glycerol palmitostearate and mixture thereof, preferably glycerol dibehenate, glycerol palmitostearate and mixture thereof, and most preferably with glycerol dibehenate. More preferably the lubricant is a mixture of magnesium stearate and/or sucrose stearate with a lubricant selected from the group consisting of glycerol dibehenate, glycerol monostearate, glycerol palmitostearate and mixture thereof, preferably glycerol dibehenate, glycerol palmitostearate and mixture thereof, most preferably with glycerol dibehenate.

Preferably the step of tablet conditioning is performed with the use of pan coater, preferably equipped with the perforated drum, tray dryer, microwave dryer or any suitable equipment in which tablets which are subject to the process of the present invention can be placed at a temperature above 40°C, preferably from 45°C to 70°C, more preferably from 50°C to 65°C, most preferably from 50°C to 60°C.

In one embodiment of the process of the present invention the step of tablet conditioning is performed with the use of pan coater equipped with the perforated drum under the following conditions: inlet air volume from 50 m³/h to 2000 m³/h, preferably from 50 m³/h to 800 m³/h, more preferably from 50 m³/h to 150 m³/h, inlet air temperature from 45°C to 70°C, preferably from 50°C to 65°C, more preferably from 50°C to 60°C, exhaust air temperature from 35°C to 65°C, preferably from 40°C to 60°C, more preferably from 50°C to 55°C, pan speed from 2 rpm to 10 rpm, preferably form 2 rpm to 7 rpm, more preferably from 2 rpm to 3 rpm.

In another embodiment of the process of the present invention the tablet which is subject to the process of the present invention comprises a binder selected from the group consisting of hydroxypropyl methylcellulose, hydroxypropylcellulose, polyvinylpyrrolidone, carboxymethylcellulose sodium; a filler/diluent selected from the group consisting of lactose monohydrate, lactose anhydrous, microcrystalline cellulose, mannitol, sorbitol; a disintegrant selected from the group consisting of Croscarmellose sodium, sodium starch glycolate, low susbstituted hydroxypropyl cellulose, pregelatinized starch; a glidant selected from the group consisting of colloidal silicon dioxide and/or a lubricant selected from the group consisting of hydrogenated castor oil, magnesium stearate, talc, glycerol dibehenate, glycerol monostearate, glycerol palmitostearate, stearic acid.

In further embodiment of the process of the present invention tablet which is subject to the process of the present invention is coated with the use of a coating agent which is selected from the group consisting of hydroxyethyl cellulose, hydroxypropyl cellulose, hydroxypropyl methylcellulose, polyvinyl alcohol.

In addition, present invention relates to a tablet obtained by the process and comprising the features as described above and the use of that tablet in the treatment or prophylaxis of thrombosis or thromboembolism and related diseases, preferably when co-administered with acetylsalicylic acid, more preferably in treatment or prophylaxis of acute myocardial infarction, unstable angina, arterial thromboembolism, transient ischemic attack, heart surgery, Kawasaki disease, percutaneous coronary intervention, stroke thrombosis.

The term tablet conditioning as used in the patent specification is to be understood as an exposure of uncoated or coated, preferably coated tablets to specified conditions of temperature.

Certain specific aspects and embodiments of the invention will be explained in more detail with reference to the following examples, being provided only for purpose of illustration, and it is to be understood that the present invention should not be deemed to be limited there to.

### Example 1

| | [wt-%/tablet] |
|---|---|
| Prasugrel maleate | 6,5 |
| Microcrystalline cellulose (PH 102) | 28,8 |
| Croscarmellose sodium (Ac-di-sol) | 2,5 |
| Mannitol (100 SD) | 57,7 |
| Hydroxypropyl methylcellulose (Methocel E4M) | 2,5 |
| Glycerol dibehenate (Compritol 888ATO) | 2,0 |

The weighed amount of prasugrel maleate, microcrystalline cellulose, croscarmellose sodium, mannitol and hydroxypropyl methylcellulose were sieved through 0.8mm sieve. Thus obtained mixture was blended for 30 minutes using a bin tumbler (Kates ML-B1109) (12 rpm). After that the weighed amount of glycerol dibehenate was added and the resulting mixture was blended for additional 10 minutes (12 rpm). The resulting mixture was compressed into a tablet with a single punch tablet press (Korsch XP 1) with the use of oblong, biconcave, 11 mm long and 5.25 mm wide punches. The resulting tablets had the weight of 200 mg per tablet before coating. These tablets were then coated with an aqueous suspension of cosmetic coating consisting of hydroxypropyl methylcellulose, lactose monohydrate, titanium dioxide, triacetin, talc, iron oxide yellow and iron oxide red (Opadry II Orange 32K23005^{®}), using a drum coater (Glatt GC 300EX) with the following conditions: inlet air temperature 50 to 55°C, exhaust air temperature 44 - 47°C, pan speed from 2 to 10 rpm, inlet air volume of 150 m3/h. After coating the tablets were kept in the drum coater for 30 minutes under the following conditions: inlet air temperature 60°C, exhaust air temperature 50-55°C, inlet air volume of 150 m3/h, drum speed of 2 rpm.

### Example 2

| | [wt-%/tablet] |
|---|---|
| Prasugrel maleate | 6,5 |
| Microcrystalline cellulose (PH 102) | 29,2 |
| Croscarmellose sodium (Ac-di-sol) | 2,5 |
| Mannitol (100 SD) | 58,3 |
| Hydroxypropyl methylcellulose (Methocel E4M) | 2,5 |
| Magnesium stearate | 1,0 |

Procedure identical to that of Example 1 was applied with an exception that instead of glycerol dibehenate magnesium stearate was added. The resulting tablets had the weight of 200 mg per tablet before coating. These tablets were then coated with the use of the same coating agent and coating conditions as in Example 1. Also the same tablet conditioning parameters as in Example 1 was applied.

### Example 3

| | [wt-%/tablet] |
|---|---|
| Prasugrel maleate | 6,5 |
| Microcrystalline cellulose (PH 102) | 28,7 |
| Croscarmellose sodium (Ac-di-sol) | 2,5 |
| Mannitol (100 SD) | 57,7 |
| Hydroxypropyl methylcellulose (Methocel E4M) | 2,5 |
| Glycerol dibehenate (Compritol 888ATO) | 2,0 |
| Magnesium stearate | 0,1 |

Procedure identical to that of Example 1 was applied with an exception that glycerol dibehenate was added with magnesium stearate. The resulting tablets had the weight of 200 mg per tablet before coating. These tablets were then coated with the use of the same coating agent and coating conditions as in Example 1. Also the same tablet conditioning parameters as in Example 1 was applied.

### Example 4

| | [wt-%/tablet] |
|---|---|
| Prasugrel maleate | 6,5 |
| Microcrystalline cellulose (PH 102) | 28,8 |
| Croscarmellose sodium (Ac-di-sol) | 2,5 |
| Mannitol (100 SD) | 55,7 |
| Hydroxypropyl methylcellulose (Methocel E4M) | 2,5 |
| Glycerol dibehenate (Compritol 888ATO) | 2,0 |
| Sucrose stearate | 2,0 |

Procedure identical to that of Example 1 was applied with an exception that sucrose stearate was added with glycerol dibehenate. The resulting tablets had the weight of 200 mg per tablet before coating. The tablets were then coated with the use of the same coating agent and coating conditions as in Example 1. Also the same tablet conditioning parameters as in Example 1 was applied.

### Example 5

| | [wt-%/tablet] |
|---|---|
| Prasugrel maleate | 6,5 |
| Microcrystalline cellulose (PH 102) | 25,8 |
| Croscarmellose sodium (Ac-di-sol) | 2,5 |
| Mannitol (100 SD) | 55,7 |
| Hydroxypropyl methylcellulose (Methocel E4M) | 2,5 |
| Glycerol dibehenate (Compritol 888ATO) | 2,0 |
| Sucrose stearate | 5,0 |

Procedure identical to that of Example 1 was applied with an exception that sucrose stearate was added with glycerol dibehenate. The resulting tablets had the weight of 200 mg per tablet before coating. Thus obtained tablets were then coated with the use of the same coating agent and coating conditions as in Example 1. Also the same tablet conditioning parameters as in Example 1 was applied.

### Example 6

| | [wt-%/tablet] |
|---|---|
| Prasugrel maleate | 6,5 |
| Microcrystalline cellulose (PH 102) | 25,8 |
| Low-substituted Hydroxypropyl cellulose (ShinEtsu) | 4,0 |
| Mannitol (100 SD) | 54,2 |
| Hydroxypropyl methylcellulose (Methocel E4M) | 2,5 |
| Glycerol dibehenate (Compritol 888ATO) | 2,0 |
| Sucrose stearate | 5,0 |

Procedure identical to that of Example 1 was applied with an exception that instead of croscarmellose sodium low-substituted hydroxypropyl cellulose was used and sucrose stearate was added with glycerol dibehenate. The resulting tablets had the weight of 200 mg per tablet before coating. Thus obtained tablets were then coated with the use of the same coating agent and coating conditions as in Example 1. Also the same tablet conditioning parameters as in Example 1 was applied.

### Comparative Example 7

| | [wt-%/tablet] |
|---|---|
| Prasugrel maleate | 6,5 |
| Microcrystalline cellulose (PH 102) | 28,8 |
| Croscarmellose sodium (Ac-di-sol) | 2,5 |
| Mannitol (100 SD) | 57,7 |
| Hydroxypropyl methylcellulose (Methocel E4M) | 2,5 |
| Glycerol dibehenate (Compritol 888ATO) | 2,0 |

Procedure identical to that of Example 1 was applied. The resulting tablets had the weight of 200 mg per tablet before coating. Thus obtained tablets were then coated with the use of the same coating agent and coating conditions as in Example 1. After coating the tablets were kept in the drum coater for 15 minutes under the following conditions: inlet air temperature 40°C, exhaust air temperature 39-41°C, inlet air volume of 150 m3/h, drum speed of 2 rpm.

### Comparative Example 8

| | [wt-%/tablet] |
|---|---|
| Prasugrel hydrochloride (form B2) | 5,5 |
| Microcrystalline cellulose (PH 102) | 22,0 |
| Croscarmellose sodium (Ac-di-sol) | 2,0 |
| Mannitol (100 SD) | 66,0 |
| Hydroxypropyl cellulose (Klucel LF) | 2,5 |
| Glycerol dibehenate (Compritol 888ATO) | 2,0 |

Procedure identical to that of Example 1 was applied with an exception that instead of prasugrel maleate prasugrel hydrochloride was used, instead of hydroxypropyl methylcellulose hydroxypropyl cellulose was used. The resulting tablets had the weight of 100 mg per tablet before coating. These tablets were then coated with the use of the same coating agent and coating conditions as in Example 1. After coating the tablets were kept in the drum coater for 15 minutes under the following conditions: inlet air temperature 40°C, exhaust air temperature 39-41°C, inlet air volume of 150 m3/h, drum speed of 2 rpm.

In the all examples the used ratio [wt-%/tablet], i.e. weight percent per tablet, was calculated based on the weight of the tablet before coating.

The resulting tablets as obtained in Examples 1 and comparative examples 7 and 8 were packed into Alu/Alu blisters under nitrogen atmosphere. All the tablets were subject to stability tests (40°C/75%RH). The results of the tests are shown in Table 1.

**Table 1 Results of the stability tests (40°C/75%RH)**

| | Example 1 | Example 7 Comparative | Example 8 Comparative |
|---|---|---|---|
| Impurity level time 0 [%] | 0.07 | 0.07 | 0.50 |
| Impurity level after 1 month [%] | 0.41 | 0.63 | 0.75 |
| Impurity level after 3 months [%] | 0.49 | 1.28 | 1.27 |

The level of impurity was tested with a use of HPLC method under the following conditions:

| | |
|---|---|
| Column: | C18 (Inertsil ODS-2 150 A 150x4,6 mm, 5 µm) |
| Mobile phase: | phase A: buffer pH 2,9 |
| | phase B: acetonitrile: water 90:10 (V/V) |
| Flow rate: | 1,0 ml/min; |
| Detection: | 235 nm; |
| Injected amount: | 20 ul |
| Run time: | 60 min |
| Diluent: | acetonitrile: water 90:10 (V/V) |

As shown in Table 1 tablets prepared according to the process of the present invention (Example 1) are more stable than tablets prepared without using such process (Comparative Examples 7 and 8).

## Claims

1. Process for preparing a tablet comprising prasugrel or pharmaceutically acceptable salt thereof and one or more pharmaceutically acceptable excipients, wherein the process comprises a step of tablet conditioning at a temperature above 40°C.

2. The process according to claim 1, wherein the process comprises the step of tablet conditioning at a temperature from 45°C to 70°C.

3. The process according to claim 1 or 2, wherein the process comprises the step of tablet conditioning at a temperature from 50°C to 65°C.

4. The process according to any preceding claim, wherein the process comprises the step of tablet conditioning at a temperature from 50°C to 60°C.

5. The process according to any preceding claim, wherein the duration of the step of tablet conditioning ranges from 5 minutes to 3 hours.

6. The process according to any preceding claim, wherein the duration of the step of tablet conditioning ranges from 10 minutes to 2 hours.

7. The process according to any preceding claim, wherein the duration of the step of tablet conditioning ranges from 15 minutes to 1 hour.

8. The process according to any preceding claim, wherein the tablet comprises prasugrel base, prasugrel hydrochloride, prasugrel hydrobromide or prasugrel maleate, more preferably prasugrel hydrochloride, prasugrel hydrobromide or prasugrel maleate, most preferably prasugrel hydrochloride or prasugrel maleate.

9. The process according to any preceding claim, wherein at least one of the one or more pharmaceutically acceptable excipients is selected from the group consisting of glycerol dibehenate, glycerol monostearate, glycerol palmitostearate and a mixture thereof, preferably from the group consisting of glycerol dibehenate, glycerol palmitostearate and a mixture thereof, and most preferably is glycerol dibehenate.

10. The process according to claim 9, wherein glycerol dibehenate, glycerol monostearate, glycerol palmitostearate or a mixture thereof is used as a lubricant.

11. The process according to claim 9 or 10, wherein glycerol dibehenate, glyceryl monostearate, glyceryl palmitostearate or a mixture thereof is used in an amount of 0.1% to 15%, more preferably 0.2% to 8%, most preferably 0.5% to 5% based on the total weight of the uncoated tablet.

12. The process according to any preceding claim, wherein the step of tablet conditioning is performed after tablet coating.

13. A tablet obtained by the process as described in any of the claims 1 to 12.

14. The tablet according to claim 13 for use in the treatment or prophylaxis of thrombosis or thromboembolism and related diseases, preferably co-administered with acetylsalicylic acid.
